# EUROPEAN PATENT SPECIFICATION

(11) **EP 2 692 924 B1**
(45) Date of publication and mention of the grant of the patent: **13.12.2017**
(21) Application number: 13178661.8
(22) Date of filing: 31.07.2013
(51) Int. Cl.: D04H 3/013, B32B 5/26, D04H 1/54, D04H 5/06, D04H 1/498, D04H 1/485, D04H 5/03, D04H 1/492, D04H 3/14, D04H 3/11, D04H 1/49, B29C 65/00, D06C 23/04, D04H 3/011

(54) **Process of making nonwoven calendered fabrics and calender**
Methode zur Herstellung kalandrierter Vliestoffe und Kalander
Procédé de fabrication des non-tissés calandrés et calandre

(30) Priority: 31.07.2012 IT MI20121340
(43) Date of publication of application: 05.02.2014
(73) Proprietor: Suominen Corporation, 00180 Helsinki (FI)
(72) Inventor: Polosa, Gianluca, FI-00180 HELSINKI (FI); Pedoja, Roberto, FI-00180 HELSINKI (FI)
(74) Representative: Seppo Laine Oy

(56) References cited:
- EP-A1- 0 557 678
- EP-A1- 1 524 350
- EP-A1- 1 939 341
- WO-A1-01/12427
- WO-A1-99/14415
- WO-A1-2006/011167
- WO-A1-2006/127075
- WO-A1-2010/039579
- WO-A1-2012/130414
- DE-A1- 10 004 448
- US-A- 4 451 520
- US-A- 5 208 098
- US-A- 5 244 482
- Albrecht, Fuchs, Kittelmann: "Nonwoven Fabrics", 1 January 2003 (2003-01-01), Wiley-VCH, Weinheim, XP002691349, ISBN: 3-527-30406-1 * page 191 *

## Description

The present invention relates to the field of non-woven textile products and is applied to the manufacture of nonwoven fabrics, particularly general nonwoven products, for various applications such as household use, personal and hygienic care. In particular, the present invention is applied to the manufacture of wipes and cleaning cloths.

Wet or impregnated wipes are of wide application in several fields. For example, nonwoven fabric cloths are used for cleaning purposes and may be impregnated with waxes or other cleaning solutions. Wet wipes are also used for personal care and may contain detergents, perfumes or even cosmetic lotions or creams.

These products are normally made from cellulose-based raw materials (typically less than 50% cellulose), such as pulp, carded viscose/lyocell airlace or spunlace, cotton and the like, which are provided with absorbent properties. High absorbent properties are necessary for the cloth to be wetted and retain a sufficiently high amount of the substance solution of the desired type (detergent, cosmetic and so on). The above raw material fibres are generally hydroentangled or firmly bonded and are wetted with the solution of detergent and/or perfume, so that they can be packaged ready for use. Pulp, viscose and lyocell absorb high amounts of water into the fibre. A stack of wet wipes with these fibres tends to be dense and its height is less than for similar wipes containing thermoplastic fibres such as polypropylene and polyester. As wet wipes are commonly packed in flexible plastic packagings, a small height of wipes will give customers a negative impression of the amount of material in the package. Additionally, a softer wipe is more appreciable by the customer due to its better feeling.

Moreover, a certain amount of polypropylene, polyester or the like is normally added to this kind of products. The presence of such thermoplastic materials in the composition of a wipe is contrary to the aim of providing biodegradable products.

Biodegradability is considered an important feature for a wet wipe. The same nature of these products implies that they should be flushable. Therefore, it is normally required to a flushable product to be biodegradable.

However, biodegradability is not the only requirement that should be ideally fulfilled when providing a flushable wipe. As waste waters are normally made to pass through pumps, particularly when they must be moved against the gravity force, for example when they reach waste treatment plants, the material should also be water dispersible to avoid that such pumps are blocked by the solid mass. Improved water dispersibility can be achieved while sacrificing the strength of the material. Biodegradability implies a long period of exposure to environmental conditions to be exercised, but water dispersibility is needed in a short time. While looking at the known products on the market, a good balance between water dispersibility and mechanical strength is still an open need in this field.

It should be considered that a mechanical stress on a sheet of nonwoven material develops along two preferential directions that are perpendicular one to the other: the MD (Machine Direction) and the CD (Cross Direction). The MD is the direction along which the material is processed: a high strength along this direction avoids that the sheet is torn during the production of the wipes. The CD direction conversely is the direction along which the wipe is dispensed by the packaging, i.e. the direction along which the customer pull the wipe out, as required by the current packaging processes. A good strength along this direction is essential as well. It is therefore important that a good balance between MD and CD strengths is obtained. This problem too is far to be solved by the prior art products.

It is therefore an object of the present invention to provide a nonwoven fabric material that is biodegradable.

It is a further object of the invention to provide a nonwoven fabric material that is water dispersible to such an extent that it can be used as a flushable product.

Another object of the invention is to provide a nonwoven fabric material having improved mechanical properties and in particular a good balance between MD and CD strength.

A still further object of the invention is to provide a nonwoven fabric material having a good softness and an appreciable height even when wet.

Another object of the invention to provide wet wipes having a nice appearance and a balanced MD/CD strength ratio and that in addition are flushable.

More particularly, the objects of the invention are processes for making the above nonwoven fabric material.

Further characteristics and the advantages of this invention will be better understood from the following detailed description of some embodiments thereof, which are provided by way of non-limiting examples wherein:

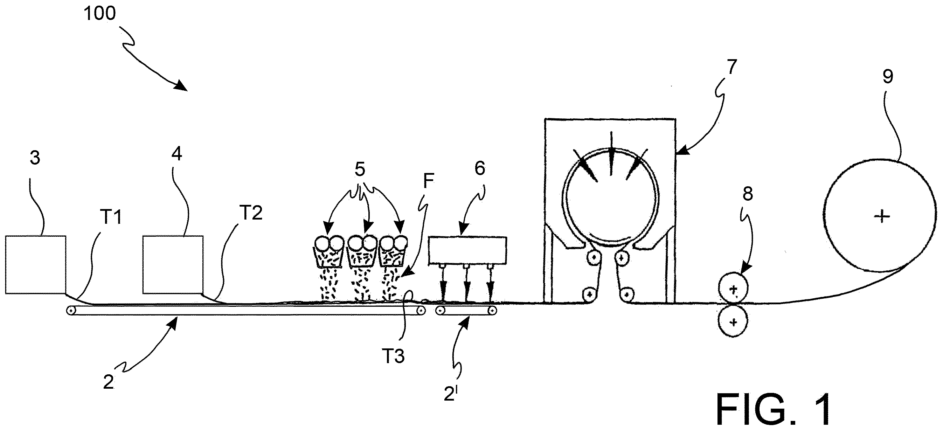

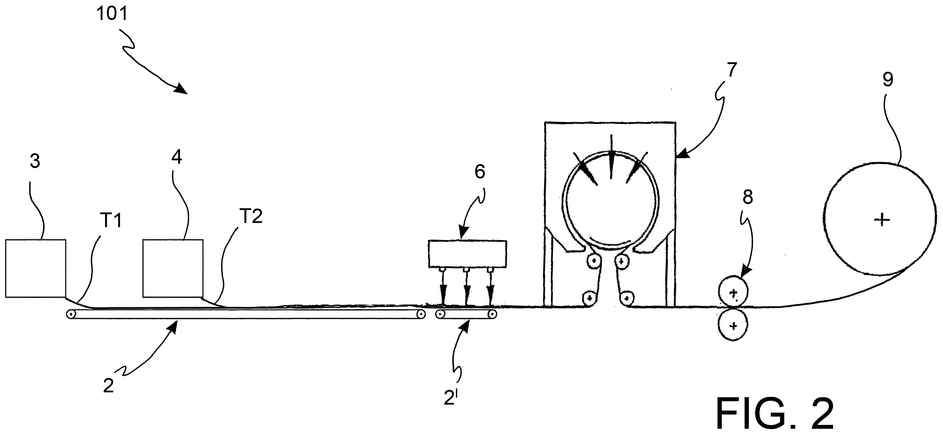

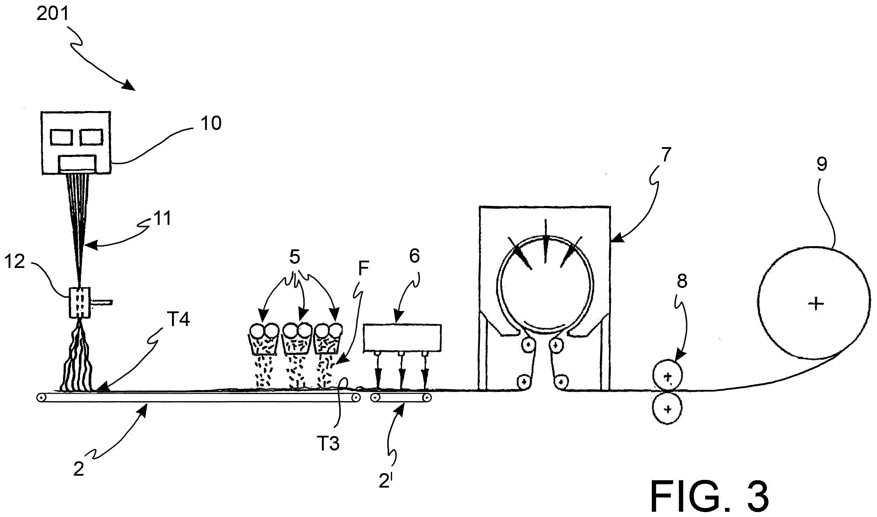

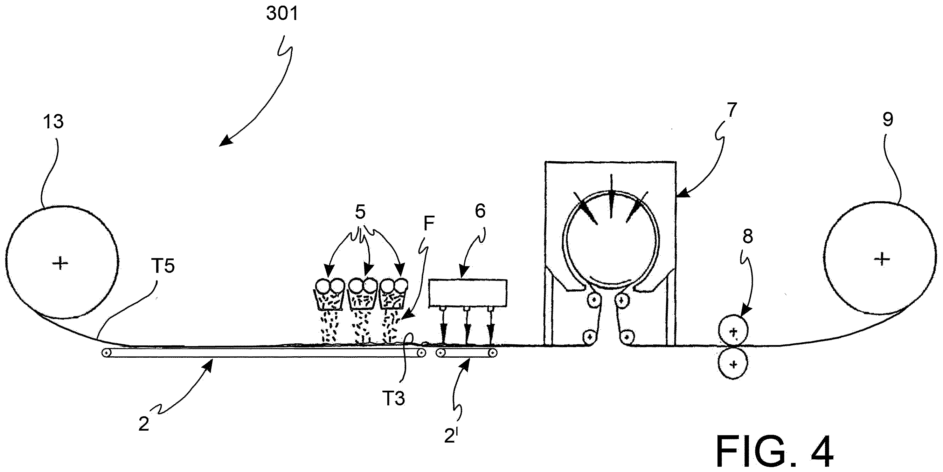

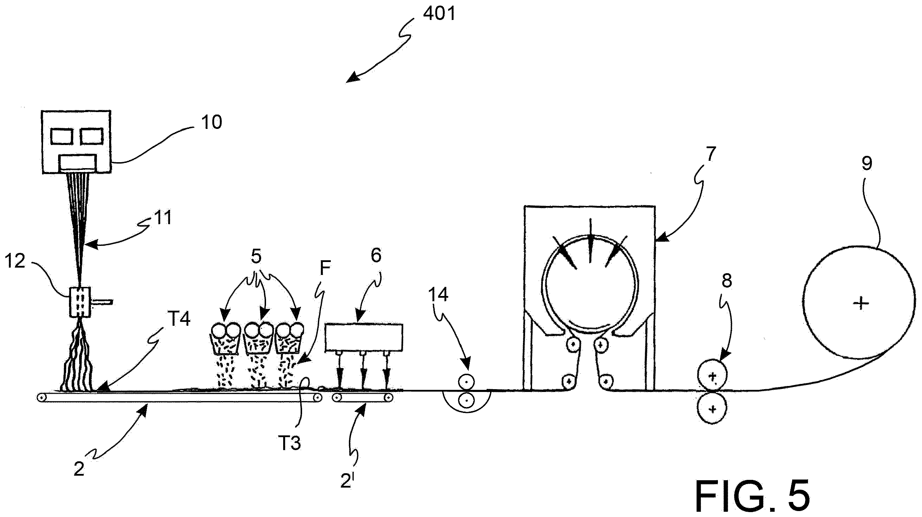

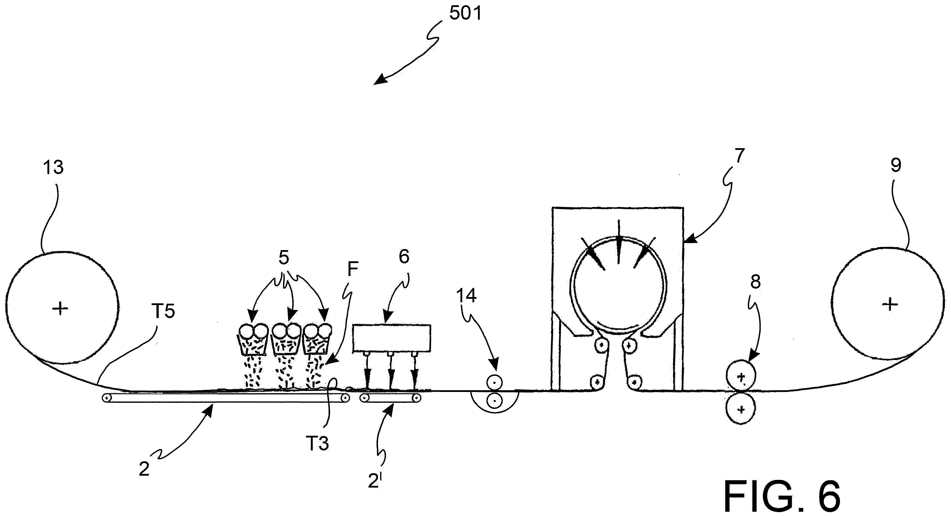

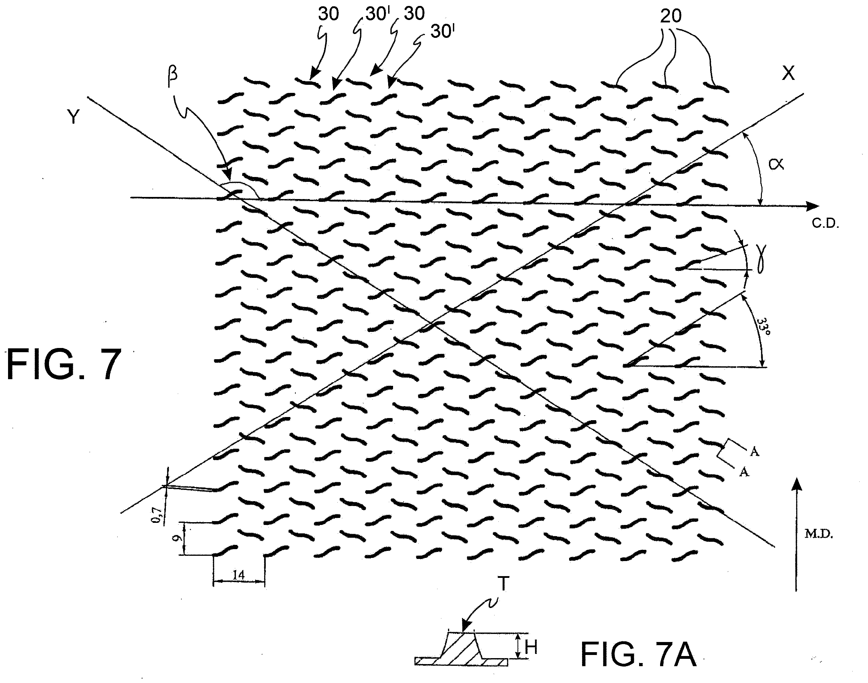

Figure 1 illustrates by way of a schematic view a first embodiment of the process of the invention; Figure 2 illustrates by way of a schematic view a second embodiment of the process of the invention; Figure 3 illustrates by way of a schematic view a third embodiment of the process of the invention; Figure 4 illustrates by way of a schematic view a fourth embodiment of the process of the invention; Figure 5 illustrates by way of a schematic view a fifth embodiment of the process of the invention; Figure 6 illustrates by way of a schematic view a sixth embodiment of the process of the invention; Figure 7 illustrates a calendering pattern according to the invention;
Figure 7A illustrates the section along the direction A-A of a particular of the engraving pattern of figure 7;
Figure 8 illustrates an engraving pattern according to the prior art that is provided as a comparison. Figure 9 illustrates another calendering pattern according to the invention;
Figure 9A illustrates the section along the direction A-A of a particular of the engraving pattern of figure 9;
Figure 10 illustrates still another calendering pattern according to the invention;
Figure 10A illustrates the section along the direction A-A of a particular of the engraving pattern of figure 9;
Figure 11 illustrates still another calendering pattern according to the invention;
Figure 11A illustrates the section along the direction A-A of a particular of the engraving pattern of figure 9.

In certain embodiments, the nonwoven fabric material produced according to the invention is a material comprising at least one layer of long fibres or of continuous filaments of a biodegradable material.

With the term "biodegradable material" it is meant either a biodegradable material as such or a compostable material.

In certain embodiments, the nonwoven fabric material produced according to the invention is a multi-layered material comprising at least one layer of long fibres or of continuous filaments of a biodegradable material and at least one layer of fibers of a water-absorbent material.

The amount of long fibres in the material of the invention may vary between 20% and 100% by weight.

In certain embodiments, the nonwoven fabric material of the invention is a multi-layered material comprising at least one layer of long fibres or of continuous filaments of a biodegradable material and at least one layer of short fibres or shortcut fibres of the same or of a different material. With the term "long fibres" fibres having a length that may vary from about 4 mm to about 60 mm, preferably from 15 to 40 mm, are meant.

With the term "short fibres" fibres having a length that may vary from substantially 0, i.e. powder, to 2.5 mm, preferably from 1 to 2 mm, are meant.

With the term "shortcut fibres" fibres having a length that may vary from about 5 to about 10 mm are meant.

With the term "continuous filament" a filament of a polymer obtained by the spunbond technique is generally intended. Continuous filaments will preferably have a diameter between 0.5 dtex and 6.7 dtex, preferably a diameter of between 0.9 and 2.5 dtex.

Non-exhaustive examples of long fibres material are viscose, lyocell, polylactic acid (PLA), optionally as a bicomponent fibre, biopolymers from cereals or plant fibres and mixtures thereof.

The term "bicomponent fibre" means a fibre obtained by coextrusion of two different polymers. They are also called "conjugate fibres". Non-limiting examples of bicomponent filament configurations include sheath-core (concentric or eccentric), side-by-side (equal or unequal portions), and islands-in-a-sea.

Non-exhaustive examples of short fibres material are cellulose pulp, cotton and mixtures thereof.

Non-exhaustive examples of shortcut fibre material are viscose, lyocell and synthetic fibres. With the term "synthetic fibres" fibres of polyester and polypropylene are exemplary meant. Non-exhaustive examples of continuous filaments material are continuous filaments of polylactic acid (PLA).

In certain embodiments, the nonwoven fabric material of the invention contains an amount of polylactic acid greater than 5% by weight, preferably greater than 10% by weight, even more preferably greater than 30% by weight.

In certain embodiments, the nonwoven fabric material produced according to the invention contains an amount of long fibres material comprised in the range of 40% to 60% by weight. In some of these embodiments, the amount of cellulose pulp in the nonwoven fabric material is comprised in the range of 40% to 60% by weight.

In certain embodiments, the nonwoven fabric material produced according to the invention contains a total amount of long fibres material and cotton of about 100% by weight (no cellulose pulp is contained).

In certain embodiments, the nonwoven fabric material of the invention contains a total amount of 100% of long fibre material.

In certain embodiments, the nonwoven fabric material produced according to the invention contains an amount of continuous filaments of spunbond PLA, or of a web of a biopolymer as defined above, comprised between about 40% and about 50% by weight, the remaining part being cellulose pulp.

In certain embodiments, the nonwoven fabric material produced according to the invention contains an amount of continuous filaments of spunbond PLA, or of a web of a biopolymer as defined above, comprised between about 40% and about 50% by weight and it is further provided with a hydrophilic coating, which is obtained by treatment of the nonwoven with wetting additives.

The wetting additives are those commonly employed in the field, such as cationic, anionic or non-ionic surfactants.

In certain embodiments, an anionic surfactant is used as a wetting agent. The said anionic surfactant is preferably an alkyl-polyglucoside ester. In one embodiment, the said alkyl polyglucoside ester is selected from disodium cocopolyglucose sulphosuccinate, disodium cocopolyglucose citrate, sodium cocopolyglucose tartrate or mixtures thereof. These surfactants are known with the trademark EUCAROL® AGE SS, EUCAROL® AGE EC and EUCAROL® AGE ET, respectively, and are commercially available from LAMBERTI spa. In the course of testing, this additive exhibited a great efficacy in terms of giving absorption capacity to the final product with a low dosage. In fact, the low dosage addition of additives leads to improved quality products as compared with those products obtained with high dosages, as will be discussed in detail below.

The basis weight of the nonwoven fabric material produced according to_the invention is in the range of between about 20 and about 60g/m². In particular, basis weight of between 30 and 60 g/m² is preferred, a basis weight of between 40 and 50 g/m² being the most preferred. However, when the nonwoven fabric material is provided with a hydrophilic coating, its basis weight will preferably be in the range of between 20 and 40 g/m².

In certain embodiments, the nonwoven fabric material produced according to the invention is a web structure that comprises more than one web, preferably 3 to 9 webs. With a number of webs in the range specified above, a more isotropic textile structure, and accordingly a maximized spatial layout of the fibres or filaments is achieved, which results into a maximized fabric-water contact surface. Thereby, the water droplets are adsorbed by the structure within the small spaces resulting from the random distribution of the fibres or filaments.

In certain embodiments, the nonwoven fabric material is a tri-layered material comprising one internal layer of said water-absorbent, flushable material, and one upper and lower layer of said long fibres or continuous filaments of a biodegradable material, in order to create a sandwich structure.

The current nonwoven fabric material can be produced by means of a process whose general steps are:
a) Laying on a support at least one layer of a biodegradable material in the form of long fibres or continuous filaments;
b) Laying on the said support or on the layer of step a) at least one layer of water-absorbent, flushable material;
c) Hydroentangling the said layers and drying the hydroentangled material;
d) Calendering the dried material of step c) to create a calendered pattern, which process is able to provide a balanced ratio of MD/CD average tensile strength to the nonwoven fabric material, the said balanced ratio of MD/CD average tensile strength being in the range of 1:1 and 4:1.

The long fibres or continuous filaments of step a) are as defined above.

The said spunbond filaments can be produced in line with the formation of the nonwoven fabric material or alternatively are made on a separate production line.

The said spunbond filaments may be produced through extrusion by spinnerets of the above defined polymer materials so as to form continuous filaments. These filaments, on output from the spinnerets, are hit by a jet of compressed air that causes the elongation and the electrostatic charging thereof such as to cause a mutual repulsion causing them to fall randomly onto a support (i.e. a conveyor belt). The continuous filament may be obtained by a spinning process by means of 1- to 5-orifices, preferably 23 orifices, spinner.

In certain embodiments, step b) comprises laying a material in the form of short fibres as defined above.

The hydroentanglement step c) is a conventional process that allows obtaining a so called spunlace web structure. This process provides for entanglement of the web fibres, moving on a perforated or patterned screen, by means of high pressurized water jets. The water pressure generally increases from the first to the last injectors, pressures up to 2200 psi are used to direct the water jets onto the web. The impinging of the water jets on the web causes the entanglement of the fibres. Exhaust water is removed by vacuum from the screen, to avoid flooding of the web. The free water being entrapped among the fibres is then eliminated by means of drying.

The drying step is typically performed in a drying oven. The temperature within the oven depends on the speed at which the product passes there through and is to be adjusted such that complete evaporation of the water is ensured. For example, if a product with 55 g/m² grammage is typically produced at 150 m/min, the temperature within the oven is to be set at about 120°C for complete water evaporation.

The calendering step is an essential step in the present invention. The calender has the basic function to partially bond the fibres/filaments composing the nonwoven and cutting the long fibres in order to weaken the strength of the material

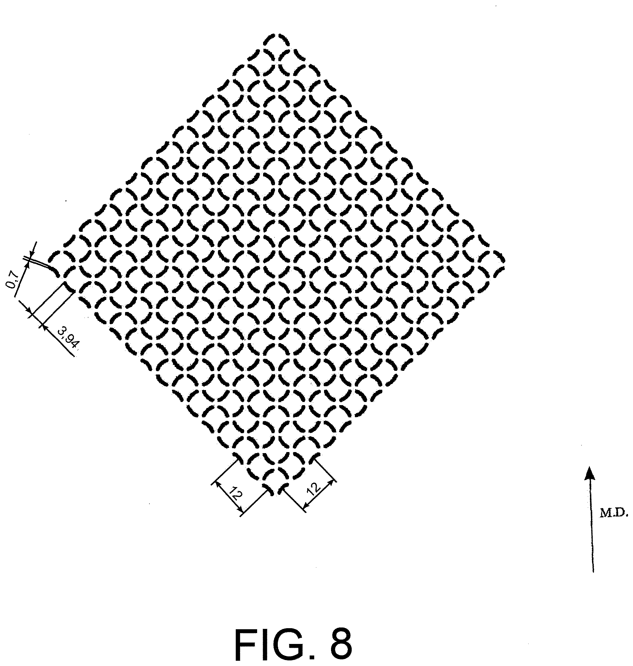

One of the two rollers, in the calender, is engraved, i.e. it has ribs in the form of dots or dashes evenly alternating with grooves. The shape, the distribution and the dimension of the dots and of the grooves strongly influences the mechanical properties of the material. In fact, it has been found that the calendering pattern according to the invention (see figures 7 and 7A) provides to the nonwoven fabric an improved balanced ratio of MD/CD average tensile strength if compared with a different calendering pattern according to the prior art (see figure 8).

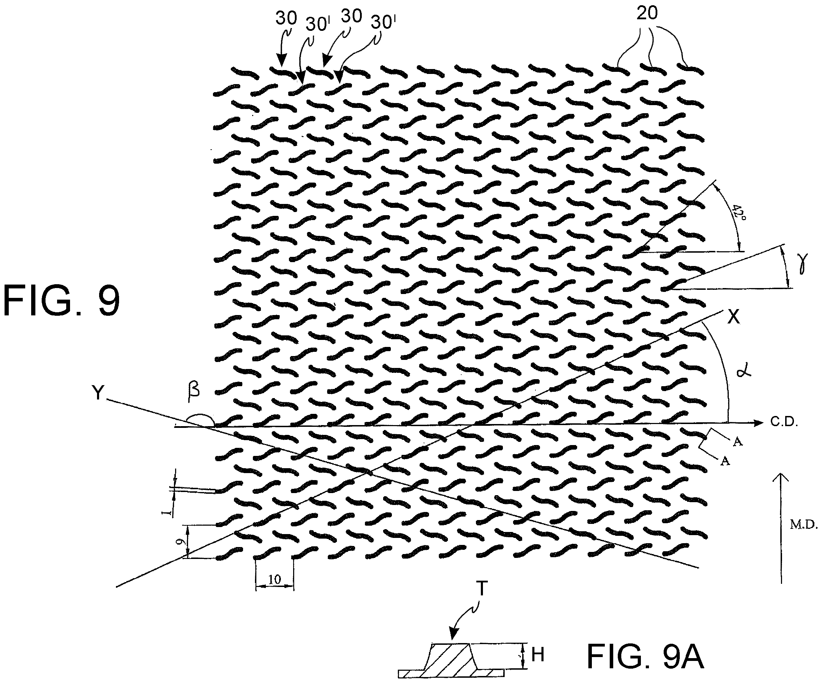

The ratio of MD/CD average tensile strength for the nonwoven fabric material of the invention is in the range 1:1 - 4:1. Preferably, the MD/CD average tensile strength ratio is in the range 1:1 and 3:1. Moreover, the MD/CD average tensile strength ratio reaches particularly advantageous values when the nonwoven fabric material is wet o moistened, so that ratio values of between 1:1 1 and 2:1 1 can be achieved. Preferred embodiments of the calendering pattern according to the invention is shown in figures 7- 7A and 9-9A.

This calendering pattern comprises a plurality of ribs 20 that are shaped like a stretched S (stretched-S-shaped). In particular, the shape of these ribs comprises a first tract having a first curvature and a second tract having a second curvature being the opposite of the first curvature, the first and the second tracts being joined by a flexion point. The sentence "a second curvature being the opposite of the first curvature" means that if the first curvature is concave, the second curvature is convex, or vice versa.

In a more preferred embodiment, the angle y formed between a line tangential to one of the two ends of a rib and a line tangential to the flexion point is from about 15° to about 25°, even more preferably about 20°.

In a preferred embodiment, the ribs have a height H comprised between 0.5 and 0.9 mm, more preferably of about 0.7 mm, a free head T with a contact surface area for the fabric of from about 4 mm² to about 10 mm², more preferably of from about 5 mm² to about 8 mm², and a distribution so that to cover 6-18%, more preferably 8-17%, of the surface of the roller.

The ribs 20 are arranged in rows along the machine direction (MD). Each row forms first modules 30 and second modules 30' in alternate alignment. Two first modules 30 that are separated by a second module 30' are defined "close modules". Analogously, two second modules 30' that are separated by a first module 30 are also defined "close modules".

In each of the first modules 30 the ribs 20 are inclined and have the same orientation, i.e. they form the same angle with the cross direction (CD). However, each first module 30 is adjacent to two second modules 30' having an opposite orientation, i.e. these latter form angles, with respect to the CD, that are supplementary to the angles formed by the first modules 30. In this way, diagonal lines X and Y are defined joining corresponding points of the ribs 20 in first modules 30 or second modules 30', which diagonal lines define the inclined orientation of the ribs 20 in such first modules 30 or second modules 30', respectively.

A first angle α is formed between the diagonal line X, defining the inclined orientation of the ribs in the first modules 30, and the cross-direction (CD), this angle α being less than 45°. In a preferred embodiment, this angle α is in the range of between 27° and 39°, more preferably between 31° and 35°, even more preferably about 33°.

Consequently, a second angle β is formed between the diagonal line Y, defining the inclined orientation of the ribs 20 in the second modules 30' and the cross-direction (CD), this second angle β being supplementary to the first angle α and being more than 135°, preferably between 153° and 141°, more preferably between 149° and 145°, even more preferably about 147°.

As said above, the distribution of the ribs 20 in the calendering pattern is an important feature. In certain embodiments, the pitch between corresponding points of adjacent ribs 20 in a same first or second module 30, 30' is comprised between 7 and 12 mm, preferably about 9 mm. Conversely, the pitch between corresponding points of ribs 20 in two close first modules 30 or in two close second modules 30' is comprised between 8 and 16 mm, preferably between 10 and 14 mm.

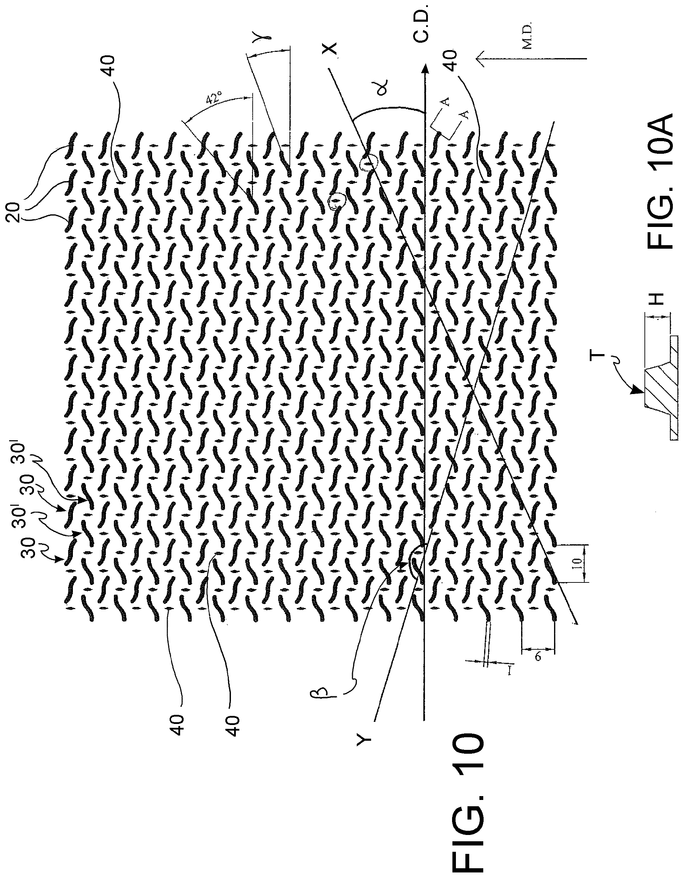

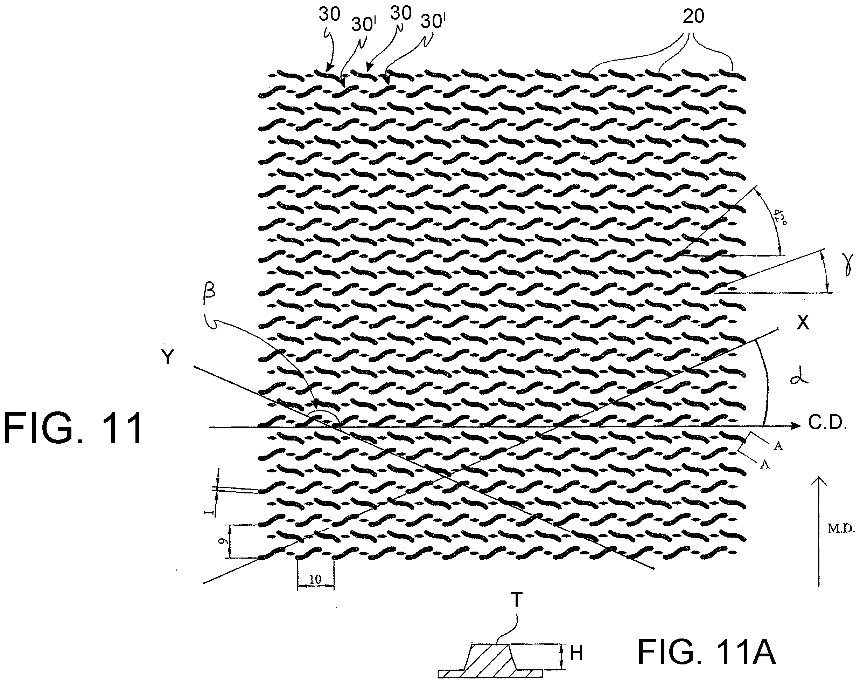

Figures 10-10A and 11-11A show alternative calendering patterns.

The pattern disclosed in figures 10-10A is the same as that of figures 9-9A, but in addition it presents a plurality of dots 40 of elongated shape, which are interposed between the ribs 20 in close rows of the first and second modules 30, 30', respectively. The elongated dots 40 are aligned along the MD direction. In consequence to the presence of such elongated dots 40, the total percentage of the surface of the roller covered by ribs 20 and dots 40 is 18-25%, preferably about 19%.

The pattern disclosed in figures 11-11A is the same as that of figures 10-10A, but the elongated dots 40 are aligned along the CD direction.

The calendering step is performed at a temperature comprised in the range from 0°C to 150°C, and at a nip pressure of in the range of 25 to 100 N/mm, preferably between 30 and 70 N/mm.

In certain embodiments, the process of the invention comprises a wetting step to confer hydrophilic properties to the material. The wetting step comprises treating the nonwoven fabric material with wetting additives as defined above. Preferably, the wetting step is performed between the hydroentanglement step and the drying step.

The wetting treatment method can be selected from those used in common practice. They are, *inter alia:*
- Spraying: the product mixture will be sprayed on the fibres;
- coating: the product mixture will be applied by a contact roller spreading the products on the web surface;
- printing: the same as coating, but using a printing machine;
- impregnation; the web structure, either already built up or in an intermediate step, is dipped in a mixture of selected products, then the excess amount is wrung by means of a mangle or similar systems;
- foam coating: a mixture of selected products is mixed with a gas (usually air) in a high speed agitator until its physical state is that of a foam, and the foam is then applied to the web by conventional coating techniques.

The dosage of the wetting additive changes as a function of the fibres being used, the nonwoven features (density, fibre distribution, etc.) and desired level of adsorption (meaning the amount of adsorbed, retained water and the adsorption time, capillarity).

The nonwoven fabric material produced according to the invention may receive various additives.

Several types of additives are added to the synthetic fibres or continuous filaments by the manufacturers, such as: lubricating additives to give smoothness and easy processability; antistatic additives to prevent damaging electrostatic currents that may degrade the product, or at worst, reduce the productivity of the machine; anti-foam additives to avoid that foam may be generated, especially upon the hydro-entanglement step.

In certain embodiments, the inventive process comprises one or more of the following final steps further to the ones described above, in order to increase or add additional characteristics to the end product: coloring or finishing of a chemical nature as the anti-pilling treatment, improvement of flame proof properties, substantially mechanical treatments such as napping, sanforizing, emerizing and the like.

The process will comprise usually a final step of winding the nonwoven fabric material to make a fabric bobbin.

The invention will be now further described by means of exemplary embodiments, with reference to the attached drawings. The drawings do not show the stations for adding the above defined additional substances, however the said stations are conventional and can be positioned at any point along the production line, preferably before the last bonding step.

In figure 1 it is shown a first embodiment of a production line 100 for manufacturing the current material.

On a transporting means 2, typically a conveyor belt, a layer T1 of long fibres as defined above is deposited in form of a carded web by a feeding line 3. A second layer T2 of short fibres (i.e. cotton fibres) is fed in form of a carded web by a second feeding line 4.

Subsequently, water-absorbent, short fibres F, typically cellulose pulp fibres, are laid through suitable dispensing means 5 to form a layer T3.

The web structure so created comprises from 40 to 60% by weight of layers T1 and T2 taken together and from 60 to 40% by weight of water-absorbent, short fibres. After the deposition of said water-absorbent, short fibres, the layered material is passed on a second transporting means 2' through hydroentangling means 6 of the conventional type. The bonded nonwoven fabric is then dried passing through drying means 7.

The dried nonwoven fabric is subjected to calendering by means of the calender 8 which has the calendering pattern depicted in figure 7.

Finally, the bonded nonwoven fabric material is wound on a receiving bobbin 9.

In figure 2 it is shown a second embodiment of a production line 101 for manufacturing the current material.

On a transporting means 2, typically a conveyor belt, a layer T1 of long fibres as defined above is deposited in form of a carded web by a feeding line 3. A second layer T2 of short fibres (i.e. cotton fibres) is fed in form of a carded web by a second feeding line 4.

The sum of the amounts of layers T1 and T2 is about 100% by weight of the nonwoven fabric.

The layered material is then passed on a second transporting means 2' through hydroentangling means 6 of the conventional type.

The bonded nonwoven fabric is then dried passing through drying means 7.

The dried nonwoven fabric is subjected to calendering by means of the calender 8 which has the calendering pattern depicted in figure 7.

Finally, the bonded nonwoven fabric material is wound on a receiving bobbin 9.

In figure 3 it is shown a third embodiment of a production line 201 for manufacturing the current material.

On a transporting means 2, typically a conveyor belt, a layer T4 of continuous filaments as defined above (i.e. PLA spunbond filaments) is deposited after being extruded by a suitable spinneret 10. The threads 11 exiting the orifices of the spinneret 10 are made to pass through a suction fan 12 positioned underneath the spinneret 10, in order to suck and convey the individual threads of extruded polymer in order to favour the bonding thereof into a single filament. Subsequently, water-absorbent, short fibres F, typically cellulose pulp fibres, are laid through suitable dispensing means 5 to form a layer T3.

The web structure so created comprises from 40 to 50% by weight of continuous filaments and from 50 to 40% by weight of water-absorbent, short fibres.

The layered material is then passed on a second transporting means 2' through hydroentangling means 6 of the conventional type.

The bonded nonwoven fabric is then dried passing through drying means 7.

The dried nonwoven fabric is subjected to calendering by means of the calender 8 which has the calendering pattern depicted in figure 7.

Finally, the bonded nonwoven fabric material is wound on a receiving bobbin 9.

In figure 4 it is shown a fourth embodiment of a production line 301 for manufacturing the current material.

On a transporting means 2, typically a conveyor belt, a web T5 of a biopolymer as defined above is deposited by a bobbin 13.

Subsequently, water-absorbent, short fibres F, typically cellulose pulp fibres, are laid through suitable dispensing means 5 to form a layer T3.

The web structure so created comprises from 40 to 50% by weight of the biopolymer and from 50 to 40% by weight of water-absorbent, short fibres.

After the deposition of said water-absorbent, short fibres, the layered material is passed on a second transporting means 2' through hydroentangling means 6 of the conventional type. The bonded nonwoven fabric is then dried passing through drying means 7.

The dried nonwoven fabric is subjected to calendering by means of the calender 8 which has the calendering pattern depicted in figure 7.

Finally, the bonded nonwoven fabric material is wound on a receiving bobbin 9.

In figure 5 it is shown a fifth embodiment of a production line 401 for manufacturing the current material.

On a transporting means 2, typically a conveyor belt, a layer T4 of continuous filaments as defined above (i.e. PLA spunlaid filaments) is deposited after being extruded by a suitable spinneret 10. The threads 11 exiting the orifices of the spinneret 10 are made to pass through a suction fan 12 positioned underneath the spinneret 10, in order to suck and convey the individual threads of extruded polymer. Subsequently, water-absorbent, short fibres F, typically cellulose pulp fibres, are laid through suitable dispensing means 5 to form a layer T3.

The web structure so created comprises from 40 to 50% by weight of continuous filaments and from 50 to 40% by weight of water-absorbent, short fibres.

The layered material is then passed on a second transporting means 2' through hydroentangling means 6 of the conventional type.

The bonded web is passed through an applicator 14 to give it a hydrophilic treatment.

The bonded nonwoven fabric is then dried passing through drying means 7.

The dried nonwoven fabric is subjected to calendering by means of the calender 8 which has the calendering pattern depicted in figure 7.

Finally, the bonded nonwoven fabric material is wound on a receiving bobbin 9.

In figure 6 it is shown a sixth embodiment of a production line 501 for manufacturing the current material.

On a transporting means 2, typically a conveyor belt, a web T5 of a biopolymer as defined above is deposited by a bobbin 13.

Subsequently, water-absorbent, short fibres F, typically cellulose pulp fibres, are laid through suitable dispensing means 5 to form a layer T3.

The web structure so created comprises from 40 to 50% by weight of the biopolymer and from 50 to 40% by weight of water-absorbent, short fibres.

After the deposition of said water-absorbent, short fibres, the layered material is passed on a second transporting means 2' through hydroentangling means 6 of the conventional type. The bonded nonwoven fabric is passed through an applicator 14 to give it a hydrophilic treatment.

The bonded nonwoven fabric is then dried passing through drying means 7.

The dried nonwoven fabric is subjected to calendering by means of the calender 8 which has the calendering pattern depicted in figure 7.

Finally, the bonded nonwoven fabric material is wound on a receiving bobbin 9.

The nonwoven fabric material of the invention can be wetted or moistened with several types of substances before packaging. The term "substance" as used herein means a solution, suspension, gel, emulsion or other wet formulation of a substance for personal care, household application or topic medical use or mixtures thereof, as the case may be. Non-limiting examples of a substance for personal care are: solutions or lotions for personal hygiene and/or sanitization, skin creams, lotions or waxes, tanning creams or lotions, sunscreen formulations, insect repellent formulations, deodorants, perfumes, antibacterial, antiviral and/or antifungal formulations, make-up removing lotions or solutions and cosmetic products in general. Non-limiting examples of a substance for household application are: detergent solutions or emulsions, waxes for ceramic or wooden floor, waxes for wood furniture surfaces, surface disinfectants, antibacterial, antiviral and/or antifungal products for household use, metal polishing emulsions, solutions or creams and cleaning formulations in general.

Non-limiting examples of a substance for topic medical use are: skin disinfectants, skin antibacterial, antiviral and/or antifungal substances, cicatrizing formulations and in general any drug formulation that can be administered by topical application, or sanitizing compositions for medical facilities, appliances or devices.

In particular, when the substance is a viscous substance as may be the case with emulsions, suspensions, gels, creams or waxes, it is important that the wetting agent is particularly efficient to impart hydrophilicity to the nonwoven fabric and to favor a distribution of the substance in the wipe. In one embodiment, with these viscous substances a nonwoven treated with an alkyl-polyglucoside ester is advantageously used. More preferably, disodium cocopolyglucose solphosuccinate can be used, due to its fluidifying effect.

The current web structure can be impregnated with the said substance by any known method as the ones described above for wetting treatment, with the proviso that the impregnated nonwoven fabric product of the invention is packaged while still wet. In particular, delivery of the substance on the web by means of suitable nozzles can advantageously be used. Impregnation as well as spraying are suitable methods for solutions or lotions.

The current textile product can be for example in the form of cloths, rags and similar fabrics, wipes, etc.

The nonwoven fabric material of the invention has various advantages over the prior art materials.

First of all, the present nonwoven fabric is soft and has a bulky appearance. A good thickness of the nonwoven, particularly when the fabric is wet or moistened, is recommended because it gives the customer an attractive appearance. This features is achieved by the nonwoven fabric of the invention in particular when PLA is contained in an amount greater than 5% by weight, preferably greater than 10% by weight.

The presence of PLA in the material also allows the patterned design to be readily visible on the wet product, which is also considered as an improvement of the product appearance.

The nonwoven fabric of the invention has also very good mechanical properties. This means that the present nonwoven fabric has a good balance between tensile strength, both in the dry and in the wet state, and water dispersibility.

In certain embodiments, the use of PLA in amounts greater than 30% by weight seems to improve the mechanical properties of the fabric in the wet state.

Table I illustrates four different trials using different compositions of the material. In all cases, the fabric was manufactured by using a process as described above, like the one depicted in figure 1, where the feeding lines 3 & 4 are fed by a carded web with a blend of viscose and PLA, but without the calendering step.

Percentages are by weight.

The base weight has been measured using the test method WSP 130.1 (ISO reference 9073-1:1989).

The thickness has been measured using the test method WSP 120.6 (ISO reference 9073 -2:1995).

The MD- and CD-tensile strengths have been determined using the test method WSP 110.4 (ISO reference 9073-3:1989).

**Table I**

| | **1** | **2** | **3** | **4** |
|---|---|---|---|---|
| | **45% pulp** | **18% pulp** | **18% pulp** | **45% pulp** |
| | **36% viscose** | **64% visc.** | **18% visc.** | **18% visc.** |
| | **18% PLA** | **18% PLA** | **64% PLA** | **36% PLA** |
| Base weight (g/m²) | 54.5 | 57.4 | 55.9 | 56.5 |
| Dry Thickness | 0.51 | 0.48 | 0.56 | 0.53 |
| Wet Thickness | 0.50 | 0.50 | 0.53 | 0.47 |
| Dry MD-strength (N/5cm) | 73.8 | 106.5 | 91.4 | 81.4 |
| Dry CD-strength (N/5cm) | 21.7 | 34.1 | 31.3 | 28.3 |
| Wet MD-strength (N/5cm) | 32.8 | 48.3 | 65.6 | 50.5 |
| Wet CD-strength (N/5cm) | 11.0 | 17.6 | 21.7 | 17.9 |
| Dry MD/CD strength | 3.4 | 3.1 | 2.9 | 2.87 |
| Wet MD/CD strength | 2.98 | 2.74 | 3.02 | 2.82 |

The data reported in Table I show that passing from dry to wet fabric both the MD- and CD-tensile strength decrease. However, when the content of PLA is above 30 % by weight, this decrease is smaller and still good values of tensile strength are achieved in the wet material.

In all instances, the MD/CD tensile strength ratio is about 3 or less, which reveals a very good balance between these strengths in the two directions. As said above, this feature allows the fabric to be resistant to rips both during its manufacture (rips along MD direction) and during customer's extraction from the package (rips along CD direction).

Table II illustrates a comparison between a nonwoven fabric material with the calendering pattern of the invention (figure 7) and the same material with a calendering pattern of the prior art as shown in figure 8.

It can be seen that the invention allows achieving a MD/CD tensile strength ratio of 2.4 which is very advantageous if compared with the prior art pattern, wherein a value of 7.1 was measured.

This result is uncommon for the prior art products, particularly for the ones based on biodegradable, water-dispersible materials.

**Table II**

| | **5 (invention)** | **6 (prior art)** |
|---|---|---|
| | 60% viscose | 60% viscose |
| | 40% cell. pulp | 40% cell. pulp |
| MD-tensile strength (N/5cm) | 24.16 | 30.87 |
| CD-tensile strength (N/5cm) | 10.12 | 4.35 |
| MD/CD ratio | 2.4 | 7.1 |

In fact, the calendering pattern according to the invention involves bonding lines to be created on the material in such an extent and orientation to impart tensile resistance along all the directions. However, this does not give adverse effects regarding the water-dispersibility of the fabric, because when the material is flushed and subject to a strong mechanical stress, like in a pump turbine, the bonding lines work as breaking lines for the material, that is then cut into small pieces that can disperse easily. Therefore, the nonwoven fabric material of the invention gives indeed a favorable balance between these properties.

It will be appreciated that only particular embodiments of the present invention have been described herein, to which those skilled in the art will be able to make any and all modifications necessary for its adjustment to specific applications, without however departing from the scope of protection of the present invention as defined in the annexed claims.

## Claims

1. A process for manufacturing a nonwoven fabric material, the process comprising:
a) Laying on a support at least one layer of a biodegradable material in the form of long fibres or continuous filaments;
b) Hydroentangling the said at least one layer and drying the hydroentangled material;
c) Calendering the dried material of step b) to create on the material a calendered pattern which is able to provide a balanced ratio of MD/CD average tensile strength to the nonwoven fabric material, the said balanced ratio of MD/CD average tensile strength being in the range of 1:1 to 4:1 or in the range of 1:1 to 3:1, wherein the calendering step is performed with a calender having ribs (20) evenly alternating with grooves so that to define a calendering pattern (1) thereon;
wherein the said calendering pattern (1) of the calender comprises a plurality of ribs (20) that are arranged in rows along the machine direction (MD), each row forming first modules (30) and second modules (30') in alternate alignment, in each of the first modules (30) the ribs (20) being inclined with the same orientation, each first module (30) being adjacent to two second modules (30') having ribs (20) being inclined with an opposite orientation, so that diagonal lines (X, Y) are defined joining corresponding points of the ribs (20) in first modules (30) or second modules (30'), which diagonal lines (X, Y) define the inclined orientation of the ribs (20) in such first modules (30) or second modules (30'), respectively, and wherein a first angle α is formed between the diagonal line (X) defining the inclined orientation of the ribs (20) in the first modules (30) and the cross-direction (CD), this first angle α being less than 45°, or in the range of between 27° and 39°, or between 31° and 35°, and a second angle β is formed between the diagonal line (Y) defining the inclined orientation of the ribs (20) in the second modules (30') and the cross-direction (CD), this second angle β being supplementary to the first angle.

2. The process according to claim 1, wherein the said ribs (20) are shaped like a stretched S with a first tract having a first curvature and a second tract having a second curvature being the opposite of the first curvature, the first and the second tracts being joined by a flexion point.

3. The process according to claim 2, wherein the angle y formed between a line tangential to one of the two ends of a rib (20) and a line tangential to the flexion point is from about 15° to about 25° or about 20°.

4. The process according to any one of claims 1 to 3, wherein the ribs (20) have a height (H) comprised between 0.5 and 0.9 mm or of about 0.7 mm, a free head with a contact surface area for the fabric of from about 4 mm² to about 10 mm², or from about 5 mm² to about 8 mm², and a distribution so that to cover 6-18%, or 8-17%, of the surface of the calender.

5. The process according to any one of claims 1 to 4, wherein the pitch between corresponding points of adjacent ribs (20) in a same first or second module (30, 30') is comprised between 7 and 12 mm or is about 9 mm.

6. The process according to any one of claims 1 to 5, wherein the pitch between corresponding points of ribs (20) in two close first modules (30) or in two close second modules (30') is comprised between 8 and 16 mm, or between 10 and 14 mm.

7. The process according to any one of claims 1 to 6, comprising a step of laying on the said support or on the layer of step a) at least one layer of water-absorbent material before the hydroentanglement step b).

8. The process according to any one of claims 1 to 7, wherein the said biodegradable material in the form of long fibres is selected from viscose, lyocell, polylactic acid (PLA), optionally as a bicomponent fibre, biopolymers from cereals or plant fibres and mixtures thereof; and wherein the said biodegradable material in the form of continuous filaments are continuous filaments of polylactic acid.

9. The process according to claim 7 or 8, wherein the said water-absorbent material is selected from cellulose pulp, cotton and mixtures thereof.

10. The process according to any one of claims 1 to 9, wherein the said calendering pattern comprises a plurality of dots (40) of elongated shape, which are interposed between the ribs (20) in close rows of the first and second modules (30, 30'), respectively.

11. The process according to claim 10, wherein the elongated dots (40) are aligned along the MD direction or along the CD direction.

12. The process according to any one of claims 1 to 11, wherein the MD/CD average tensile strength ratio when the nonwoven fabric material is wet or moistened is between 1:1 and 2:1.

13. A calender wherein the calendering pattern (1) of the calender comprises a plurality of ribs (20) shaped like a stretched S with a first tract having a first curvature and a second tract having a second curvature being the opposite of the first curvature, the first and the second tracts being joined by a flexion point; said ribs being arranged in rows along the machine direction (MD), each row forming first modules (30) and second modules (30') in alternate alignment, in each of the first modules (30) the ribs (20) being inclined with the same orientation, each first module (30) being adjacent to two second modules (30') having ribs (20) being inclined with an opposite orientation, so that diagonal lines (X, Y) are defined joining corresponding points of the ribs (20) in first modules (30) or second modules (30'), which diagonal lines (X, Y) define the inclined orientation of the ribs (20) in such first modules (30) or second modules (30'), respectively, and wherein a first angle α is formed between the diagonal line (X) defining the inclined orientation of the ribs (20) in the first modules (30) and the cross-direction (CD), this first angle α being in the range of between 27° and 39°, or between 31° and 35°, and a second angle β is formed between the diagonal line (Y) defining the inclined orientation of the ribs (20) in the second modules (30') and the cross-direction (CD), this second angle β being supplementary to the first angle.

14. The calender according to claim 13, wherein the said calendering pattern comprises a plurality of dots (40) of elongated shape, which are interposed between the ribs (20) in close rows of the first and second modules (30, 30'), respectively.

## Patentansprüche

1. Verfahren zur Herstellung eines Vliesstoffs, wobei das Verfahren umfasst:
a) Legen auf einen Träger mindestens einer Lage eines bioabbaubaren Materials in Form langer Fasern oder Endlosfilamenten;
b) Wasserstrahlverfestigung der mindestens einen Lage und Trocknen des wasserverfestigten Materials;
c) Kalandrieren des getrockneten Materials von Schritt b) zur Schaffung eines kalandrierten Musters auf dem Material, das befähigt ist, ein ausgeglichenes Verhältnis von mittlerer Zugfestigkeit MD/CD für den Vliesstoff bereitzustellen, wobei das ausgeglichene Verhältnis von mittlerer Zugfestigkeit MD/CD im Bereich von 1:1 bis 4:1 oder im Bereich von 1:1 bis 3:1 liegt, wobei der Kalandrierschritt mit einem Kalander ausgeführt wird, der Rippen (20) ebenmäßig abwechselnd mit Rillen aufweist, sodass ein Kalandriermuster (1) darauf definiert wird;
wobei das Kalandriermuster (1) des Kalanders eine Vielzahl von Rippen (20) umfasst, die in Reihen entlang der Maschinenrichtung (MD) angeordnet sind, wobei jede Reihe erste Module (30) und zweite Module (30') in abwechselnder Ausrichtung bildet, wobei in jedem der ersten Module (30) die Rippen (20) mit derselben Orientierung geneigt sind, wobei jedes erste Modul (30) zu zwei zweiten Modulen (30') benachbart ist, die Rippen (20) aufweisen, die mit einer entgegengesetzten Orientierung geneigt sind, sodass diagonale Linien (X, Y) definiert werden, die entsprechende Punkte der Rippen (20) in ersten Modulen (30) oder zweiten Modulen (30') verbinden, wobei die diagonalen Linien (X, Y) die geneigte Orientierung der Rippen (20) in solchen ersten Modulen (30) bzw. zweiten Modulen (30') definieren, und wobei ein erster Winkel α zwischen der diagonalen Linie (X), die die geneigte Orientierung der Rippen (20) in den ersten Modulen (30) definiert, und der Querrichtung (CD) gebildet wird, wobei dieser erste Winkel α weniger als 45° beträgt, oder im Bereich zwischen 27° und 39°, oder zwischen 31° und 35° liegt, und ein zweiter Winkel β zwischen der diagonalen Linie (Y), die die geneigte Orientierung der Rippen (20) in den zweiten Modulen (30') definiert, und der Querrichtung (CD) gebildet wird, wobei dieser zweite Winkel β sich mit dem ersten Winkel ergänzt.

2. Verfahren nach Anspruch 1, wobei die Rippen (20) wie ein gestrecktes S geformt sind, mit einem ersten Zug, der eine erste Krümmung aufweist und einem zweiten Zug, der eine zweite Krümmung aufweist, die entgegengesetzt zur ersten Krümmung ist, wobei die ersten und die zweiten Züge durch einen Wendepunkt verbunden sind.

3. Verfahren nach Anspruch 2, wobei der Winkel y, der zwischen einer Linie tangential zu einem der zwei Enden einer Rippe (20) und einer Linie tangential zu dem Wendepunkt gebildet wird, von etwa 15° bis etwa 25° oder etwa 20° beträgt.

4. Verfahren nach einem der Ansprüche 1 bis 3, wobei die Rippen (20) eine Höhe (H), die zwischen 0,5 und 0,9 mm oder etwa 0,7 mm beträgt, einen freien Kopf mit einem Kontaktflächengebiet für das Textil von etwa 4 mm² bis etwa 10 mm² oder von etwa 5 mm² bis etwa 8 mm² und eine Verteilung aufweisen, sodass 6-18 % oder 8-17 % der Oberfläche des Kalanders zu bedecken sind.

5. Verfahren nach einem der Ansprüche 1 bis 4, wobei der Abstand zwischen entsprechenden Punkten der benachbarten Rippen (20) in einem gleichen ersten oder zweiten Modul (30, 30') zwischen 7 und 12 mm beträgt oder etwa 9 mm ist.

6. Verfahren nach einem der Ansprüche 1 bis 5, wobei der Abstand zwischen entsprechenden Punkten von Rippen (20) in zwei nahen ersten Modulen (30) oder in zwei nahen zweiten Modulen (30') zwischen 8 und 16 mm oder zwischen 10 und 14 mm beträgt.

7. Verfahren nach einem der Ansprüche 1 bis 6, umfassend einen Schritt zum Legen auf den Träger oder auf die Lage von Schritt a) mindestens einer Lage von wasserabsorbierendem Material vor dem Wasserverfestigungsschritt b).

8. Verfahren nach einem der Ansprüche 1 bis 7, wobei das bioabbaubare Material in Form langer Fasern aus Viskose, Lyocell, Polymilchsäure (PLA), gegebenenfalls als eine Zweikomponenten-Faser, Biopolymeren aus Getreide oder Pflanzenfasern und Gemischen davon ausgewählt ist; und wobei das bioabbaubare Material in Form von Endlosfilamenten Endlosfilamente von Polymilchsäure sind.

9. Verfahren nach Anspruch 7 oder 8, wobei das wasserabsorbierende Material aus Zellstoff, Watte und Gemischen davon ausgewählt ist.

10. Verfahren nach einem der Ansprüche 1 bis 9, wobei das Kalandriermuster eine Vielzahl von Punkten (40) von länglicher Form umfasst, die zwischen die Rippen (20) in nahen Reihen der ersten bzw. zweiten Module (30, 30') eingeschoben sind.

11. Verfahren nach Anspruch 10, wobei die länglichen Punkte (40) entlang der MD-Richtung oder entlang der CD-Richtung ausgerichtet sind.

12. Verfahren nach einem der Ansprüche 1 bis 11, wobei das mittlere MD/CD Zugfestigkeitsverhältnis, wenn der Vliesstoff nass oder befeuchtet ist, zwischen 1:1 und 2:1 beträgt.

13. Kalander, wobei das Kalandriermuster (1) des Kalanders eine Vielzahl von Rippen (20), geformt wie ein gestrecktes S mit einem ersten Zug, der eine erste Krümmung aufweist, und einem zweiten Zug, der eine zweite Krümmung aufweist, die entgegengesetzt von der ersten Krümmung ist, umfasst, wobei die ersten und die zweiten Züge durch einen Wendepunkt verbunden sind; wobei die Rippen in Reihen entlang der Maschinenrichtung (MD) angeordnet sind, jede Reihe erste Module (30) und zweite Module (30') in abwechselnder Ausrichtung bildet, in jedem der ersten Module (30) die Rippen (20) mit derselben Orientierung geneigt sind, jedes erste Modul (30) benachbart zu zwei zweiten Modulen (30') ist, mit Rippen (20), die mit einer entgegengesetzten Orientierung geneigt sind, sodass diagonale Linien (X, Y) definiert werden, die entsprechende Punkte der Rippen (20) in ersten Modulen (30) oder zweiten Modulen (30') verbinden, die diagonalen Linien (X, Y) die geneigte Orientierung der Rippen (20) in solchen ersten Modulen (30) bzw. zweiten Modulen (30') definieren, und wobei ein erster Winkel α zwischen der diagonalen Linie (X), die die geneigte Orientierung der Rippen (20) in den ersten Modulen (30) definiert und der Querrichtung (CD) gebildet wird, wobei dieser erste Winkel α im Bereich zwischen 27° und 39° oder zwischen 31° und 35° liegt, und ein zweiter Winkel β zwischen der diagonalen Linie (Y), die die geneigte Orientierung der Rippen (20) in den zweiten Modulen (30') definiert und der Querrichtung (CD) gebildet wird, wobei dieser zweite Winkel β mit dem ersten Winkel ergänzend ist.

14. Kalander nach Anspruch 13, wobei das Kalandriermuster eine Vielzahl von Punkten (40) länglicher Form umfasst, die zwischen die Rippen (20) in nahen Reihen der ersten bzw. zweiten Module (30, 30') eingeschoben sind.

## Revendications

1. Procédé pour la fabrication d'une matière à base de textile non tissé, le procédé comprenant le fait de :
a) déposer sur un support au moins une couche d'une matière biodégradable sous la forme de longues fibres ou de filaments continus ;
b) soumettre à un hydroliage ladite au moins une couche et sécher la matière soumise à un hydroliage ;
c) calandrer la matière séchée de l'étape b) pour créer sur la matière un motif calandré qui est capable de conférer à la matière à base de textile non tissé un rapport équilibré de la résistance moyenne à la traction MD/CD, ledit rapport équilibré de la résistance moyenne à la traction MD/CD se situant dans la plage de 1:1 à 4:1 ou dans la plage de 1:1 à 3:1, dans lequel l'étape de calandrage est mise en oeuvre avec une calandre possédant des nervures (20) qui alternent de manière uniforme avec des rainures de façon à y définir un motif de calandrage (1) ;
dans lequel ledit motif de calandrage (1) de la calandre comprend plusieurs nervures (20) qui sont disposées en rangs dans le sens machine (MD), chaque rang formant des premiers modules (30) et des seconds modules (30') dans un alignement alterné, dans chacun des premiers modules (30) les nervures (20) étant inclinées avec la même orientation, chaque premier module (30) étant adjacent à deux seconds modules (30') dont les nervures (20) sont inclinées avec une orientation opposée, d'une matière telle que des lignes diagonales (X, Y) sont définies qui joignent des points correspondants des nervures (20) dans des premiers modules (30) ou des seconds modules (30'), lesdites lignes diagonales (X, Y) définissant l'orientation inclinée des nervures (20) dans lesdits premiers modules (30) ou lesdits seconds modules (30'), respectivement, et dans lequel un premier angle α est formé entre la ligne diagonale (X) définissant l'orientation inclinée des nervures (20) dans les premiers modules (30) et la direction transversale (CD), ce premier angle α étant inférieur à 45°, ou étant situé dans la plage entre 27° et 39°, ou entre 31° et 35°, et un second angle β est formé entre la ligne diagonale (Y) définissant l'orientation inclinée des nervures (20) dans les seconds modules (30') et la direction transversale (CD), ce second angle β étant supplémentaire au premier angle.

2. Procédé selon la revendication 1, dans lequel lesdites nervures (20) prennent la forme d'un S étiré comprenant une première étendue possédant une première courbure et une seconde étendue possédant une seconde courbure opposée à la première courbure, la première et la seconde étendue étant jointes par un point de flexion.

3. Procédé selon la revendication 2, dans lequel l'angle y formé entre une ligne tangentielle à une des deux extrémités d'une nervure (20) et une ligne tangentielle au point de flexion représente d'environ 15° à environ 25° ou environ 20°.

4. Procédé selon l'une quelconque des revendications 1 à 3, dans lequel les nervures (20) possèdent une hauteur (H) comprise entre 0,5 et 0,9 mm ou d'environ 0,7 mm, une tête libre comprenant une aire de surface de contact pour le tissu d'environ 4 mm² à environ 10 mm², ou d'environ 5 mm² à environ 8 mm², et une distribution de façon à couvrir de 6 à 18 %, ou de 8 à 17 %, de la surface de la calandre.

5. Procédé selon l'une quelconque des revendications 1 à 4, dans lequel le pas entre des points correspondants de nervures adjacentes (20) dans un même premier ou second module (30, 30') est compris entre 7 et 12 mm ou s'élève à environ 9 mm.

6. Procédé selon l'une quelconque des revendications 1 à 5, dans lequel le pas entre des points correspondants de nervures (20) dans deux premiers modules proches (30) ou dans deux seconds modules proches (30') est compris entre 8 et 16 mm, ou entre 10 et 14 mm.

7. Procédé selon l'une quelconque des revendications 1 à 6, comprenant une étape consistant à déposer sur ledit support ou sur la couche de l'étape a) au moins une couche d'une matière absorbant l'eau avant l'étape d'hydroliage b).

8. Procédé selon l'une quelconque des revendications 1 à 7, dans lequel ladite matière biodégradable sous la forme de longues fibres est choisie parmi la viscose, le lyocell, l'acide polylactique (PLA), de manière facultative sous la forme d'une fibre à deux composants, des biopolymères de fibres céréalières ou végétales, ainsi que leurs mélanges; et dans lequel ladite matière biodégradable sous la forme de filaments continus représente des filaments continus d'acide polylactique.

9. Procédé selon la revendication 7 ou 8, dans lequel ladite matière absorbant l'eau est choisie parmi de la pâte de cellulose, du coton, et leurs mélanges.

10. Procédé selon l'une quelconque des revendications 1 à 9, dans lequel ledit motif de calandrage comprend plusieurs points (40) de configuration allongée, qui sont intercalés entre les nervures (20) des premiers et seconds modules (30, 30') en rangs serrés, respectivement.

11. Procédé selon la revendication 10, dans lequel les points allongés (40) sont alignés dans le sens MD ou dans la direction CD.

12. Procédé selon l'une quelconque des revendications 1 à 11, dans lequel le rapport de la résistance moyenne à la traction MD/CD, lorsque la matière à base de textile non tissé est mouillée ou humidifiée, se situe entre 1:1 et 2:1.

13. Calandre dans laquelle le motif de calandrage (1) de la calandre comprend plusieurs nervures (20) prenant la forme d'un S étiré comprenant une première étendue possédant une première courbure et une seconde étendue possédant une seconde courbure opposée à la première courbure, la première et la seconde étendue étant jointes par un point de flexion ; lesdites nervures étant disposées en rangs dans le sens machine (MD), chaque rang formant des premiers modules (30) et des seconds modules (30') dans un alignement alterné, dans chacun des premiers modules (30) les nervures (20) étant inclinées avec la même orientation, chaque premier module (30) étant adjacent à deux seconds modules (30') dont les nervures (20) sont inclinées avec une orientation opposée, d'une matière telle que des lignes diagonales (X, Y) sont définies qui joignent des points correspondants des nervures (20) dans des premiers modules (30) ou des seconds modules (30'), lesdites lignes diagonales (X, Y) définissant l'orientation inclinée des nervures (20) dans lesdits premiers modules (30) ou lesdits seconds modules (30'), respectivement, et dans lequel un premier angle α est formé entre la ligne diagonale (X) définissant l'orientation inclinée des nervures (20) dans les premiers modules (30) et la direction transversale (CD), ce premier angle α se situant dans la plage entre 27° et 39°, ou entre 31° et 35°, et un second angle β est formé entre la ligne diagonale (Y) définissant l'orientation inclinée des nervures (20) dans les seconds modules (30') et la direction transversale (CD), ce second angle β étant supplémentaire au premier angle.

14. Calandre selon la revendication 13, dans laquelle ledit motif de calandrage comprend plusieurs points (40) de configuration allongée, qui sont intercalés entre les nervures (20) des premiers et seconds modules (30, 30') en rangs serrés, respectivement.
